# EUROPEAN PATENT SPECIFICATION

(11) **EP 1 880 702 B1**
(45) Date of publication and mention of the grant of the patent: **01.02.2012**
(21) Application number: 07112654.4
(22) Date of filing: 17.07.2007
(51) Int. Cl.: A61H 1/00, A63B 22/18, A63B 26/00, A61B 5/103, A61B 5/11

(54) **Motorized platform for the therapeutic treatment of patients**
Motorisierte Plattform zur therapeutischen Behandlung von Patienten
Plate-forme motorisée pour le traitement thérapeutique de patients

(30) Priority: 20.07.2006 IT RM20060387
(43) Date of publication of application: 23.01.2008
(73) Proprietor: Universita'Degli Studi di Roma "La Sapienza", 00185 Roma (IT); Ospedale Pediatrico Bambino Gesù IRCCS, 00165 Rome (IT)
(72) Inventor: CAPPA, Paolo Università degli Studi di Roma "La Sapienza", 00185, Roma (IT); PATANE', Fabrizio Università degli Studi di Roma "La Sapienza", 00185, Rome (IT)
(74) Representative: Papa, Elisabetta

(56) References cited:
- DE-A1- 10 019 162
- US-A- 6 063 046
- US-A1- 2003 199 374
- US-A1- 2004 144 288
- US-B1- 6 774 885

## Description

The present invention refers to a device for the assessment of a subject's motor abilities and for the treatment of any disorder - in particular postural disorders deriving, e.g., from neurodegenerative syndromes - by a platform movable according to three rotational degrees of freedom.

A reduction in posture and walking adjustment abilities is an excellent functional indicator, both for early analysis and control of neurovegetative syndromes evolution. Therefore, systems capable of representing concretely the evolution of the case history and of the entailed therapeutic treatment constitute relevant instruments of diagnosis and care.

In particular, the so-called "functional training", regardless of the patient's specific pathology and age, should evolve the target motor function, opposing the consequences induced by neurobiological alterations. Above all in "slow" evolutions, only a training aimed at the patient's specificities can succeed to keep up certain performance levels, opposing the disruption of distributed neural maps, and to restructure the latter by using the spared tissue.

Therefore, as mentioned above, an objective qualitative and quantitative functional assessment of individual motor abilities is necessary to define the functional state of the action systems and to compare among the same performances at different times, above all during rehabilitating pre- and post-treatment.

The evolution of knowledge on the postural system is steering toward the manufacturing of training instruments based on the programming of perturbations to postural stability, - both during the standing position and the walking - to measure the subject's abilities to compensate for perturbations in a predictive manner, when those are made predictable, and in real time, when unpredictable, to keep and attain the aim of the task undertaken.

Therefore, in order to represent concretely and also to recover any motor deficit, systems have been advanced, both in a paediatric context and for adults, and for several pathologies (neurodegenerative ones, ictuses, tumours, etc.), which, via the setting of known perturbations and the detection of the effects those cause in the subject, be capable of assessing the motor abilities and, if necessary, of effectively contributing to the rehabilitation of the same subject.

To date, the instruments commonly used to carry out a rehabilitation treatment for postural stability are comprised of swinging platforms of various types, manually and non-manually operated ones.

In particular, the known motorised platforms, called "Stewart platforms", have six degrees of freedom, obtained by using as actuators six independently controlled electric cylinders connecting a movable platform to a stationary base; the control system varies the length of the electric cylinders in a coordinated manner, so as to obtain a change in the position and orientation of the movable platform. This solution, though having reduced technological complexity for the overall kinematic motion, entails however the drawback that the electric cylinders cannot have a length, in the configuration of maximum retraction, shorter than about twice its travel. Therefore, the height dimensions of these devices are constrained by the longitudinal dimension of the actuators. Hence, since in order to attain sufficiently wide RPJ (roll-pitch-yaw) rotations high-travel actuators have to be used, the minimum heights of the platforms from the ground are accordingly always greater and can compromise an outpatient use thereof, as well as necessary safety levels required for the patient.

In more general terms, one of the major drawbacks of marketed platforms is constituted by the relevant dimensions and considerable weight, as well as the need to provide important safety measures to safeguard patient's safety during assessment or rehabilitation.

Moreover, always in general terms, many of the commercially available platforms allow neither a specific adjustment for the patient's pathology, nor adjustability to the rehabilitation course envisaged by the medical staff.

US 2003/0199374 discloses a machine for assessing and improving a user's proprioception, which includes a platform that may have three rotational degrees of freedom, a ball joint centrally-mounted to the platform and at least two reciprocating actuators causing the platform to tilt.

DE 100 19 162 discloses a six-degree-of-freedom moving platform actuated by three linear drives arranged in parallel configuration and connected to the platform by telescopic rods.

The technical problem set and solved by the present invention is to provide a device for motor assessment and rehabilitation based on a revolving (swinging) platform, allowing to overcome the drawbacks mentioned above with reference to the current state of the art.

Such a problem is solved by a device according to claim 1.

Preferred features of the present invention are present in the dependent claims thereof.

The present invention provides several relevant advantages. The main advantage lies in that it has reduced dimensions and weight and allows a positioning of the swinging platform at moderate heights above the ground, to the advantage of: (a) safety of the same subject and (b) reduced dimensions, facilitating its installing in laboratories.

A further advantage of the invention, particularly in the preferred embodiments that will be illustrated hereinafter, is to allow an objective assessment of the degree of disability and the adoption of a training procedure based on the imposition of a perturbation known and repeated over time.

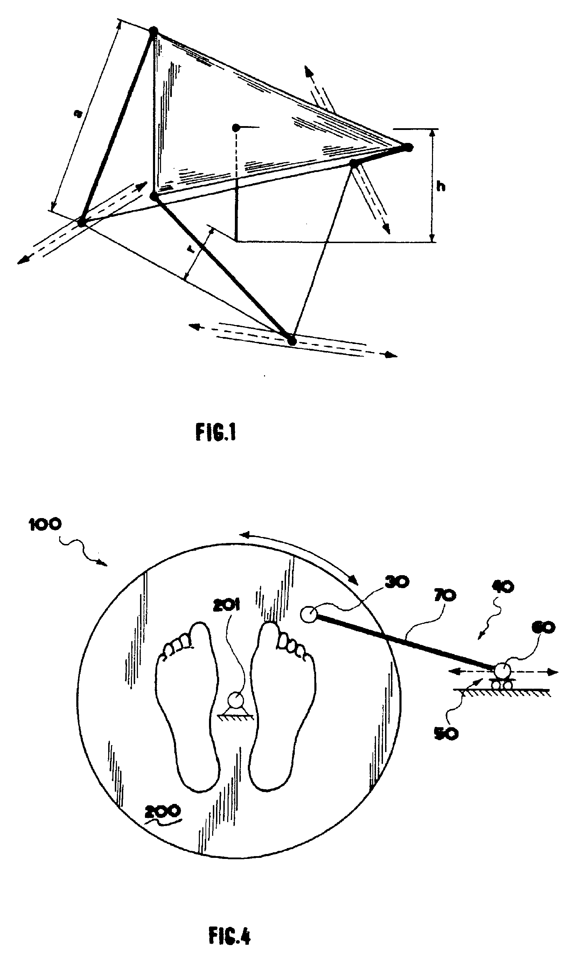

Other advantages, features and the operation steps of the present invention will be made apparent by the following detailed description of some embodiments thereof, given by way of example and not for limitative purposes. Reference will be made to the figures of the annexed drawings, wherein the example of Fig. 4 is not covered by the claims.
- Figure 1 shows a broad depiction of the kinematic diagram of a preferred embodiment of the device for motor assessment and treatment according to the present invention;
- Figure 2 shows a perspective view of an embodiment of the device corresponding to the kinematic diagram of Figure 1;
- Figure 3 shows a perspective view of the platform of Figure 2 during normal use with a subject; and
- Figure 4 shows a schematic top plan depiction of a simplified example with a single degree of freedom of the platform of Figure 1.

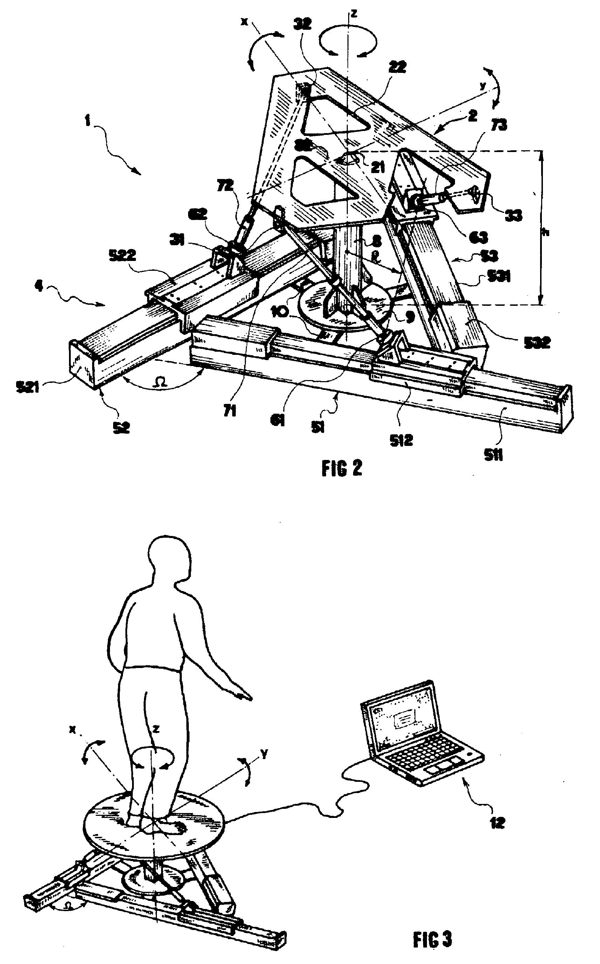

Initially referring to Figures 1 and 2, a device suitable for motor assessment and rehabilitation, in particular of postural disorders deriving from neurodegenerative syndromes, is generally denoted by 1.

More in detail, the device 1 comprises at least one movable platform or footboard 2, apt to support a subject in erect posture, provided with three rotational degrees of freedom, associable to the so-called RPJ rotation angles. In the present example the platform 2 has a substantially equilateral triangle-shaped plan configuration and it is revolving about a fixed point 21 substantially corresponding to its own geometric centroid. Preferably, the platform 2 has one or more weight-lightening holes 22. Substantially in correspondence of or near to its own vertexes, the platform 2 is connected by respective ball joints 31, 32 and 33 to moving means, generally denoted by 4, associated with said three rotational degrees of freedom. Such moving means 4 comprises in particular three alike and independent linear actuators, respectively denoted by 51, 52 and 53.

Each linear actuator 51, 52, 53 comprises a respective stationary slide guide, respectively denoted by 511, 521 and 531, arranged horizontal and in particular substantially parallel to or lying onto the floor surface; onto said guide there can slide, always in a direction that can be deemed parallel to the floor surface, a respective shoe-like element, respectively denoted by 512, 522 and 532, thereby forming a prismatic coupling between each guide 511-531 and the respective shoe element 512-532.

Each shoe element 512-532 is associated with an electric motor (for simplicity's sake not shown) that sets it in motion.

Moreover, each shoe element 512-532 has on the upper part thereof a respective ball joint, respectively denoted by 61, 62 and 63, for connection to a respective rod, 71, 72 and 73, connected each with the respective ball joint 31-33 associated with the movable platform 2.

In a preferred embodiment, the guides 511-531 are arranged so that between two adjacent guides there be defined an angle Ω preferably equal to about 120°.

Therefore, on the basis of the hereto-described arrangement, by sliding on the stationary guides 511-531 the shoe elements 512-532 cause, via the pairs of ball joints 61-31, 62-32 and 63-33, the rotation of the movable platform 2 about the centre of rotation 21.

In particular, as mentioned above, in the present example the movable platform 2 is provided with one rotary degree of freedom about a substantially vertical axis *z* and two rotational degrees of freedom about two respective transversal axes *x, y* orthogonal thereamong. As it is shown in Figure 2, for simplicity's sake said reference system *x, y, z* has been selected with an origin in correspondence of the fixed point 21. It is provided adjusting means allowing to vary at least two geometric parameters of the device 1, and specifically the height of the movable platform 2 from the ground, definable as distance *h* of the centre of rotation 21 from the floor surface, and a radius of said platform 2, definable as distance R between the on-ground foot of said height *h* and the line joining two adjacent rotoidal pairs among those defined by the ball joints 61-63. Such an adjustment allows to modify the variation range of the three angles of rotation RPJ.

Said adjusting means comprises a collapsible adjusting mechanism associated with each of the connecting elements 71-73.

In the present example, in which the platform 2 has only rotational degrees of freedom, said platform is supported by a substantially vertical upright 8, extending substantially from the centre of rotation 21 to the level of the floor surface, i.e. the level of the linear actuators 51-53. The upright 8 is fixed, in correspondence of the floor surface, to a stationary support base 9, in turn fixed to the guides 511-531 by suitable side arms 10. The supporting upright 8 is instead connected to the movable platform 2 by means of a ball joint 82.

The upright 8 is apt to discharge onto the ground the weight of the subject resting on the movable platform 2. Thus, the moving means 4, and in particular the actuators 51-53, the associated motors, the transmission rods 71-73 and the ball joints 31-33 and 61-63, should be dimensioned so as to allow the rotation of the platform 2 and compensate for the dynamic vertical components of the patient's weight not perfectly centred with respect to the upright 8.

The upright 8 has variable length, so as to allow said variation of the parameters *h* and *R* characterising the kinematics of the device 1. E.g., the upright 8 may be collapsible.

As it is schematically shown in Figure 3, the platform 1, always according to a preferred embodiment, comprises also control, data acquisition and processing means, generally denoted by 12. In particular, such means 12 comprises:
- means apt to drive and manage the motion set to the platform 2 by the moving means 4, which may provide to the movable platform 2 itself a perturbation known and adjustable depending on the user's age and/or the specific pathology and vary the law of motion set during a same training cycle;
- measuring means, apt to detect the forces acting on the movable platform 2, i.e. forces exerted by the subject, and this preferably by suitable sensors or local transducers apt to measure stresses, in particular tensile and compressive stresses, positioned onto the transmission rods 71-73; and
- means for processing said measuring.

In particular, the means for processing the measuring is apt to determine, on the basis of the instantaneous geometric configuration assumed by the platform 2 and the stresses acting onto the driving rods 71-73, the moment of rotation that the user opposes to the motion of the platform 2 itself. Such a moment equals to a measuring of the ability of the subject's balance system to adjust to external postural perturbations.

Concerning the operation modes of the device 1 described hereto, initially this can suitably be prearranged by varying in the above-illustrated manner the height *h* from the ground and said radius R of the movable platform 2 depending on the variation ranges of the three angles of rotation RPJ to be mainly used.

E.g., it is preferable to minimize both *h* and R when wishing to use platform 1 mainly for rotations in the horizontal plane about axis z (yaw angle J). On the contrary, both *h* and R have to be increased when envisaging instead a mainly swinging operation, of the "*EQUITEST*® *NeuroCom International, Inc*" (balance test) type.

Then, the law of motion of the movable platform 2 is set by an operator who may be in the lab (local configuration) or instead on other premises linked via a computer network (remote configuration) by said control means.

Once the preliminary steps have been carried out and the subject is on the movable platform 2, the control means start, either in local configuration or in remote configuration, said swinging/rotary motion of the movable platform 2; said motion will go on for a predetermined time and with predetermined modes. The control means is capable, according to the program of perturbations to postural stability selected by the operator, of varying, during the training cycle, the law of motion set to the platform 2.

During the test or training associated with said motion of the platform 2, the load cells measure stresses acting on each transmission element 71-73 and transmit the data to the means for processing the measuring. The latter means derives the moment of rotation that the user opposes to the motion of the movable platform 2.

At the elapsing of the predetermined time, or through a control directly actuated by either the operator or the patient, the movable platform 2 will cease its motion and the processing means will further process the collected data, providing an assessment of the subject's motor abilities directly in the form of a clinical report.

It will be appreciated that the device 1 is completely reconfigurable thanks to the option of adjusting said parameters *h* and R.

Moreover, by now it will be better appreciated that the device of the invention allows to set a known perturbation to a subject, scale the perturbation depending on age and the specific pathology, and vary the law of motion set.

Therefore, the device is particularly suitable for the postural rehabilitation of subjects suffering from various types of pathologies, e.g. hemiplegic or diplegic children, who however represent most of the patients usually hospitalized in Children's Neurorehabilitation Wards. Moreover, the device finds effective use also in the functional recovery of adult patients suffering from cerebral damages and in the training of elderly people.

Figure 4 shows a schematic depiction of a simplified example of the device, in this case generally denoted by 100.

The device 100 comprises a movable platform 200, in the present example substantially of circular shape, revolving about an axis substantially orthogonal thereto and passing through its geometric centre 201. In correspondence of the latter, the movable platform 200 is pivotably connected to a central pin, for simplicity's sake not depicted.

In this case as well the device 100 comprises moving means, here generally denoted by 40, including a single linear actuator 50 that may it also be of the type already described with reference to the preceding embodiment. The actuator 50 is rotatably connected, by interposition of a rod 70, to the movable platform 200; such a connection is made by two respective rotational joints 60 and 30 associated with the two ends of the driving element 70 itself. In particular, the joint 30 is arranged near to or in correspondence of the periphery of the movable platform 200.

Overall, the linear actuator 50, the driving element 70 and the movable platform 200 carry out a connecting rod/crank kinematic motion, yet of course this may be replaced by any one equivalent mechanism apt to produce a rotary motion about said vertical axis.

The device 100 considered here is contrived above all to attain an effective stress on the vestibular system.

The device of this example exhibits an intrinsic constructive simplicity, a limited variability of the input that is set to the subject and the option of entirely making remote the device in a simple and effective manner, so as to make a telerehabilitation system.

This specific aptitude to telerehabilitation use is particularly relevant, as the substantial limit of rehabilitation therapies based on the use of pivoting platforms lies in their high cost, perspectively causing its acquisition at highly specialized wards only. However, an intensive rehabilitation treatment seldom allows to overcome deficits prior to the patient's returning home, with the entailed need to carry it on even afterwards. The development of rehabilitation-dedicated instruments based on remote control by the rehabilitation team, instruments that can be installed at the patient's dwelling, would instead allow the carrying out of the rehabilitation intervention with an adequate intensity and according to the recovery project already under way. The device of the invention is capable of setting a rehabilitative exercise that be iterated and provided with adequate sensory feedback for adjustment; it is adjustable in remote mode to a patient's specific training needs, it allows the patient, as well as the rehabilitation team, to have real-time information on the training management and the progresses attained, and allows to the rehabilitation team the customizing and the online managing of the neuromotor training.

## Claims

1. A device (1) apt to the motor assessment and rehabilitation, in particular of postural disorders, comprising:
- a movable platform (2), apt to support a subject in erect posture and having three rotational degrees of freedom, wherein the overall arrangement is such that said movable platform (2) has a fixable centre of rotation (21);
- moving means (4) for moving said movable platform (2) according to said rotational degrees of freedom,
wherein said moving means (4) comprises three linear actuators (51-53), connected to said movable platform (2) and arranged or apt to be arranged, in operative configuration of the device (1), substantially parallel to the floor surface,
which linear actuators (51-53) are arranged so that pairs of adjacent actuators (51-52, 52-53, 53-51) form an angle (Ω) equal to about 120 deg and each of which linear actuator (51-53) is connected to said movable platform (2) by a respective motion transmission element (71-73) rotatably connected to the actuator (51-53) itself and to said platform (2),
each motion transmission element (71-73) being substantially in the form of a rod, rotatably connected in correspondence of its own longitudinal ends to said movable platform (2) and to said respective actuator (51-53), respectively;
- means for adjusting the geometric configuration of the device, apt to modify the height (h) of said movable platform (2) from the ground by means of said transmission elements (71-73) having adjustable length; and
- an element (8) for supporting said movable platform (2) and apt to support the weight of the subject engaging said movable platform (2), which element (8) has a variable length, is arranged substantially in correspondence of said fixable centre of rotation (21) of said movable platform (2) and is rotatably connected to the latter.

2. The device (1) according to claim 1, wherein said movable platform (2) is rotatable about a substantially vertical axis (z).

3. The device (1) according to any of the preceding claims, wherein said fixable centre of rotation (21) corresponds to a geometric centre or centroid of said movable platform (2).

4. The device (1) according to any of the preceding claims, wherein said rotatable connection between said or each transmission element (71-73) and said platform (2) and respective actuator (51-53) is made by ball joints (31-33, 61-63).

5. The device (1) according to any of the preceding claims, wherein said transmission elements (71-73) have a collapsible structure.

6. The device (1) according to any of the preceding claims, wherein said rotatable connection between movable platform (2) and supporting element (8) is made by a ball joint (82).

7. The device (1) according to any one of the preceding claims, wherein said supporting element comprises a substantially vertical upright (8).

8. The device (1) according to any one of the preceding claims, comprising control means (12) for controlling the motion of said movable platform (2), means that can be actuated by an operator so as to set the law of motion of the movable platform (2) itself.

9. The device (1) according to the preceding claim, wherein said control means (12) is apt or allows to select the perturbation provided to said movable platform (2) depending on the subject's age and/or the specific pathology.

10. The device (1) according to any one of the preceding claims, comprising data acquisition means (12), apt to detect stresses exerted by the subject onto said movable platform (2).

11. The device (1) according to the preceding claim, wherein said data acquisition means (12) comprises sensors or transducers apt to detect stresses applied onto elements (71-73) of said moving means (4).

12. The device (1) according to the preceding claim, wherein said data acquisition means (12) is apt to detect tensile and/or compressive stresses.

13. The device (1) according to the preceding claim, wherein said processing means (12) is apt to determine, on the basis of the geometric configuration assumed by said movable platform (2) and of the measuring of stresses acting onto said platform (2) and/or said moving means (4), the moment that the subject opposes to the motion of the platform (2) itself.

14. The device (1) according to any one of the claims 8 to 13, wherein said control, acquisition and/or processing means allow a remote interaction with the device (1) itself.

## Patentansprüche

1. Vorrichtung (1), die zur Beurteilung der Bewegungsfähigkeit und zur Rehabilitation geeignet ist, insbesondere von Haltungsschäden, mit:
- einer beweglichen Plattform (2), die geeignet ist, eine Person in aufrechter Haltung zu tragen, und drei Drehfreiheitsgrade hat, wobei die Gesamtanordnung so getroffen ist, dass die bewegliche Plattform (2) einen fixierbaren Drehungsmittelpunkt (21) hat;
- einer Bewegungseinrichtung (4) zum Bewegen der beweglichen Plattform (2) gemäß den Drehfreiheitsgraden,
wobei die Bewegungseinrichtung (4) drei lineare Stellantriebe (51-53) umfasst, die mit der beweglichen Plattform (2) verbunden und angeordnet sind oder geeignet sind, angeordnet zu werden in operativer Konfiguration der Vorrichtung (1), im Wesentlichen parallel zu der Bodenfläche,
wobei die linearen Stellantriebe (51-53) so angeordnet sind, dass Paare benachbarter Stellantriebe (51-52, 52-53, 53-51) einen Winkel (Ω) bilden, der etwa gleich 120° ist, und dass jeder lineare Stellantrieb (51-53) mit der beweglichen Plattform (2) durch ein Bewegungsübertragungselement (71-73) verbunden ist, welches mit dem Stellantrieb (51-53) selbst und mit der Plattform (2) verbunden ist,
wobei jedes Bewegungsübertragungselement (71-73) im Wesentlichen die Form einer Stange hat, die an ihren eigenen Längsenden mit der beweglichen Plattform (2) bzw. mit dem zugeordneten Stellantrieb (51-53) verbunden ist;
- einer Einrichtung zum Einstellen der geometrischen Konfiguration der Vorrichtung, die geeignet ist, die Höhe (h) der beweglichen Plattform (2) über dem Boden mit Hilfe der Übertragungselemente (71-73), die eine einstellbare Länge haben, zu modifizieren; und
- einem Element (8) zum Tragen der beweglichen Plattform (2), das geeignet ist, das Gewicht der sich auf der beweglichen Plattform (2) befindlichen Person aufzunehmen, wobei das Element (8) eine variable Länge hat, im Wesentlichen in dem fixierbaren Drehungsmittelpunkt (21) der beweglichen Plattform (2) angeordnet und mit letzterer drehbar verbunden ist.

2. Vorrichtung (1) nach Anspruch 1, wobei die bewegliche Plattform (2) um eine im Wesentlichen vertikale Achse (z) drehbar ist.

3. Vorrichtung (1) nach einem der vorhergehenden Ansprüche, wobei der fixierbare Drehungsmittelpunkt (21) einem geometrischen Mittelpunkt oder dem Schwerpunkt der beweglichen Plattform (2) entspricht.

4. Vorrichtung (1) nach einem der vorhergehenden Ansprüche, wobei die drehbare Verbindung zwischen dem oder jedem Übertragungselement (71-73) der Plattform (2) und dem betreffenden Stellantrieb (51-53) durch Kugelgelenke (31-33, 61-63) gebildet ist.

5. Vorrichtung (1) nach einem der vorhergehenden Ansprüche, wobei die Übertragungselemente (71-73) einen zusammenlegbaren Aufbau haben.

6. Vorrichtung (1) nach einem der vorhergehenden Ansprüche, wobei die drehbare Verbindung zwischen der beweglichen Plattform (2) und dem tragenden Element (8) durch ein Kugelgelenk (82) gebildet ist.

7. Vorrichtung (1) nach einem der vorhergehenden Ansprüche, wobei das tragende Element ein im Wesentlichen vertikales aufrechtes Teil (8) umfasst.

8. Vorrichtung (1) nach einem der vorhergehenden Ansprüche, mit einer Steuereinrichtung (12) zum Steuern der Bewegung der beweglichen Plattform (2), eine Einrichtung, die durch eine Bedienungsperson betätigt werden kann, um so das Bewegungsgesetz der beweglichen Plattform (2) selbst einzustellen.

9. Vorrichtung (1) nach dem vorhergehenden Anspruch, wobei die Steuereinrichtung (12) geeignet ist oder erlaubt, die Störung auszuwählen, die durch die bewegliche Plattform (2) erfolgt, in Abhängigkeit von dem Alter und/oder der besonderen Pathologie der Person.

10. Vorrichtung (1) nach einem der vorhergehenden Ansprüche, mit einer Datenerfassungseinrichtung (12), die geeignet ist, Belastungen zu erfassen, die durch die Person auf die bewegliche Plattform (2) ausgeübt werden.

11. Vorrichtung (1) nach dem vorhergehenden Anspruch, wobei die Datenerfassungseinrichtung (12) Sensoren oder Wandler umfasst, die geeignet sind, Belastungen zu erfassen, die auf die Elemente (71-73) der Bewegungseinrichtung (4) ausgeübt werden.

12. Vorrichtung (1) nach dem vorhergehenden Anspruch, wobei die Datenerfassungseinrichtung (12) in der Lage ist, Zug- und/oder Druckbelastungen zu erfassen.

13. Vorrichtung (1) nach dem vorhergehenden Anspruch, wobei die Verarbeitungseinrichtung (12) geeignet ist, auf der Basis der geometrischen Konfiguration, die durch die bewegliche Plattform (2) eingenommen wird, und der Messung von Beanspruchungen, die auf die Plattform (2) und/oder die Bewegungseinrichtung (4) ausgeübt werden, das Moment zu bestimmen, mit dem die Person der Bewegung der Plattform (2) selbst entgegenwirkt.

14. Vorrichtung (1) nach einem der Ansprüche 8 bis 13, wobei die Steuer-, die Erfassungs- und /oder die Verarbeitungseinrichtung eine Fernwechselwirkung mit der Vorrichtung (1) selbst erlauben.

## Revendications

1. Dispositif (1) permettant l'évaluation et la réhabilitation motrice, en particulier pour les désordres posturaux, comprenant :
- une plate-forme mobile (2) apte à supporter un sujet en position debout et ayant trois degrés de liberté en rotation, l'agencement d'ensemble étant tel que ladite plate-forme mobile (2) a un centre de rotation réglable (21) ;
- un moyen de déplacement (4) pour mouvoir ladite plate-forme mobile (2) selon lesdits degrés de liberté en rotation,
dans lequel ledit moyen de déplacement (4) comprend trois actionneurs linéaires (51-53) connectés à ladite plate-forme mobile (2) et agencés ou aptes à être agencés, dans la configuration fonctionnelle du dispositif (1), substantiellement parallèlement à la surface du sol,
lesquels actionneurs linéaires (51-53) sont agencés de telle manière que les paires d'actionneurs adjacents (51-52, 52-53, 53-51) forment un angle (Ω) égal à environ 120 degrés et chacun des actionneurs linéaires (51-53) est connecté à ladite plate-forme mobile (2) par un élément de transmission du mouvement respectif (71-73) connecté à rotation à l'actionneur (51-53) lui-même et à ladite plate-forme (2),
chaque élément de transmission du mouvement (71-73) se présentant substantiellement sous la forme d'une tige, connectée à rotation en correspondance avec ses propres extrémités longitudinales respectivement à ladite plate-forme mobile (2) et audit actionneur respectif (51-53) ;
- un moyen pour régler la configuration géométrique du dispositif, apte à modifier la hauteur (h) de ladite plate-forme mobile (2) par rapport au sol au moyen desdits éléments de transmission (71-73) ayant une longueur réglable ; et
- un élément (8) pour supporter ladite plate-forme mobile (2) et apte à supporter le poids du sujet qui se place sur ladite plate-forme mobile (2), lequel élément (8) a une longueur variable, est placé substantiellement en correspondance avec ledit centre de rotation réglable (21) de ladite plate-forme mobile (2) et est connecté à rotation à cette dernière.

2. Dispositif (1) selon la revendication 1, dans lequel ladite plate-forme mobile (2) peut tourner autour d'un axe substantiellement vertical (z).

3. Dispositif (1) selon l'une quelconque des revendications précédentes, dans lequel ledit centre de rotation réglable (21) correspond à un centre géométrique ou centre de gravité de ladite plate-forme mobile (2).

4. Dispositif (1) selon l'une quelconque des revendications précédentes, dans lequel ladite connexion rotative entre ledit ou chaque élément de transmission (71-73) et ladite plate-forme (2) et un actionneur respectif (51-53) est réalisée par des articulations sphériques (31-33, 61-63).

5. Dispositif (1) selon l'une quelconque des revendications précédentes, dans lequel lesdits éléments de transmission (71-73) ont une structure démontable.

6. Dispositif (1) selon l'une quelconque des revendications précédentes, dans lequel ladite connexion rotative entre la plate-forme mobile (2) et l'élément de support (8) est réalisée par une articulation sphérique (82).

7. Dispositif (1) selon l'une quelconque des revendications précédentes, dans lequel ledit élément de support comprend un montant substantiellement vertical (8).

8. Dispositif (1) selon l'une quelconque des revendications précédentes, comprenant un moyen de commande (12) pour commander le mouvement de ladite plate-forme mobile (2), moyen pouvant être actionné par un opérateur afin de définir la loi de mouvement de la plate-forme mobile (2) elle-même.

9. Dispositif (1) selon la revendication précédente, dans lequel ledit moyen de commande (12) est apte à ou permet de sélectionner la perturbation fournie à ladite plate-forme mobile (2) en fonction de l'âge et/ou de la pathologie spécifique du sujet.

10. Dispositif (1) selon l'une quelconque des revendications précédentes, comprenant un moyen d'acquisition de données (12) apte à détecter les contraintes exercées par le sujet sur ladite plate-forme mobile (2).

11. Dispositif (1) selon la revendication précédente, dans lequel ledit moyen d'acquisition de données (12) comprend des capteurs ou des transducteurs aptes à détecter les contraintes appliquées sur les éléments (71-73) dudit moyen de déplacement (4).

12. Dispositif (1) selon la revendication précédente, dans lequel ledit moyen d'acquisition de données (12) est apte à détecter les contraintes de traction et/ou de compression.

13. Dispositif (1) selon la revendication précédente, dans lequel ledit moyen de traitement (12) est apte à déterminer, en se basant sur la configuration géométrique adoptée par ladite plate-forme mobile (2) et sur la mesure de contraintes agissant sur ladite plate-forme (2) et/ou ledit moyen de déplacement (4), le moment où le sujet s'oppose au mouvement de la plate-forme (2) elle-même.

14. Dispositif (1) selon l'une quelconque des revendications 8 à 13, dans lequel lesdits moyens de commande, acquisition et/ou traitement permettent une interaction à distance avec le dispositif (1) en lui-même.
